(19) Europäisches Patentamt

European Patent Office

Office européen des brevets

(11) **EP 2 864 448 B2**

(12) **NOUVEAU FASCICULE DE BREVET EUROPEEN**
Après la procédure d'opposition

(45) Date de publication et mention de la décision concernant l'opposition:
**28.08.2019 Bulletin 2019/35**

(45) Mention de la délivrance du brevet:
**06.04.2016 Bulletin 2016/14**

(21) Numéro de dépôt: **13723833.3**

(22) Date de dépôt: **18.04.2013**

(51) Int Cl.:
*C10G 29/20* (2006.01)  *C10G 29/28* (2006.01)
*C10G 45/72* (2006.01)  *B01J 8/04* (2006.01)
*C10G 3/00* (2006.01)  *C07C 303/04* (2006.01)
*C07C 1/207* (2006.01)  *C07C 1/24* (2006.01)
*C07C 2/66* (2006.01)  *C07C 2/72* (2006.01)

(86) Numéro de dépôt international:
**PCT/FR2013/050850**

(87) Numéro de publication internationale:
**WO 2013/190193 (27.12.2013 Gazette 2013/52)**

(54) **PROCEDE DE PRODUCTION DE TENSIO-ACTIFS A PARTIR DE MATIERES RENOUVE-LABLES COMPRENANT UNE ETAPE D'HYDROTRAITEMENT ET UNE ÉTAPE DE TRANSFORMATION DE PARAFFINES EN TENSIO-ACTIFS**

VERFAHREN ZUR HERSTELLUNG VON TENSIDEN AUS ERNEUERBAREN MATERIALIEN MIT EINEM HYDRODESULFURIERUNGSSCHRITT UND EINEM SCHRITT ZUR UMWANDLUNG VON PARAFFINEN IN TENSIDE

METHOD FOR PRODUCING SURFACTANTS FROM RENEWABLE MATERIALS INCLUDING A STEP OF HYDROTREATING AND A STEP OF TRANSFORMING PARAFFINS INTO SURFACTANTS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.06.2012 FR 1201760**

(43) Date de publication de la demande:
**29.04.2015 Bulletin 2015/18**

(73) Titulaire: **IFP Energies nouvelles**
**92500 Rueil-Malmaison (FR)**

(72) Inventeur: **CHAPUS, Thierry**
**F-69001 Lyon (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
EP-A1- 1 741 768    EP-A1- 2 226 375
EP-A1- 2 428 548    FR-A1- 2 967 687
US-A1- 2006 287 562    US-A1- 2007 281 875

• KUBICKOVÁ ET AL: "Hydrocarbons for diesel fuel via decarboxylation of vegetable oils", CATALYSIS TODAY, vol. 106, no. 1-4, 2005, pages 197-200, XP027834544,

EP 2 864 448 B2

**Description**

L'invention concerne un procédé de production de composés tensio-acifs, à partir d'une charge issue de sources renouvelables telles que les huiles et graisses d'origine végétale ou animale, comprenant une étape d'hydrotraitement de ladite charge pour produire des hydrocarbures paraffiniques et en particulier n-paraffiniques, et une étape de transformation desdits hydrocarbures paraffiniques en composés tensio-acifs utilisables notamment comme détergents.

Le contexte international actuel est marqué d'abord par le besoin de trouver des substituts aux produits pétroliers, pour les applications carburants mais aussi pour des applications production de produits chimiques. Parmi ces produits chimiques, on peut citer les tensio-actifs ou "surfactants" pour "surface active agent" selon la terminologie anglo-saxonne, qui sont caractérisés par leur capacité à abaisser la tension superficielle entre deux surfaces. Lesdits tensio-actifs sont des molécules amphiphiles, c'est à dire présentant deux parties de polarité différente, l'une lipophile et apolaire, l'autre hydrophile, c'est-à-dire miscible dans l'eau et polaire. Les propriétés remarquables des tensio-actifs expliquent leur utilisation pour un grand nombre d'applications, comme détergents, agents de solubilisation, agents moussants, agents mouillants, agents dispersants ou encore agents émulsifiants.

Dans ce contexte, la recherche de nouvelles charges issues de sources renouvelables constitue un enjeu d'une importance croissante. Parmi ces charges, on peut citer par exemple les huiles végétales (alimentaires ou non) ou issues d'algues et les graisses animales.

Ces charges sont principalement composées de triglycérides et/ou d'acides gras libres et/ou d'esters, ces molécules comprenant des chaînes hydrocarbonées d'acides gras avec un nombre d'atomes de carbone allant de 4 à 25, et un nombre d'insaturations généralement compris entre 0 et 3, avec des valeurs plus élevées pour les huiles d'algues par exemple. Les charges renouvelables contiennent en outre des impuretés comme des composés azotés, et des métaux présents sous forme de phospholipides contenant des éléments comme le phosphore, le calcium, le magnésium, le potassium et le sodium. Les chaînes hydrocarbonées qui constituent lesdites charges sont essentiellement linéaires et leur longueur en terme de nombre d'atomes de carbone est généralement compris entre 4 et 25.

Il est donc intéressant de transformer ces charges renouvelables. Une approche possible est la transformation catalytique desdites charges en composés hydrocarbonés paraffiniques désoxygénés en présence d'hydrogène et en particulier par hydrotraitement.

Lors de l'hydrotraitement, les réactions subies par ladite charge contenant les triglycérides et/ou les acides gras et/ou les esters sont les suivantes:

- la réaction d'hydrogénation des insaturations des chaînes hydrocarbonées des triglycérides, des acides gras et des esters,
- les réactions de désoxygénation selon deux voies réactionnelles possibles :

   ∘ l'hydrodésoxygénation (HDO) conduisant à la formation d'eau par consommation d'hydrogène et à la formation d'hydrocarbures de nombre de carbone ($C_n$) égal à celui des chaînes d'acides gras initiales,
   ∘ la décarboxylation/décarbonylation conduisant à la formation d'oxydes de carbone (monoxyde et dioxyde de carbone : CO et $CO_2$) et à la formation d'hydrocarbures comptant un carbone en moins ($C_{n-1}$) par rapport aux chaînes d'acides gras initiales.

L'hydrogénation des insaturations des chaînes hydrocarbonées (double liaisons carbone-carbone) est fortement exothermique et l'augmentation de température provoquée par le dégagement de chaleur peut conduire à des niveaux de température où la part des réactions de décarboxylation devient davantage favorisée. Les réactions d'hydrodésoxygénation, y compris les réactions de décarboxylation, sont également des réactions exothermiques.

Un contrôle strict de la température dans la section hydrotraitement est nécessaire, puisqu'une température trop élevée présente également les inconvénients de favoriser les réactions secondaires indésirables comme la polymérisation, le craquage, le dépôt de coke et la désactivation du catalyseur. Les réactions de polymérisation peuvent conduire à une dégradation de la charge avant sa transformation selon la voie désirée. Les réactions de craquage présentent l'inconvénient de dégrader le rendement de la transformation souhaitée.

Par ailleurs, les hydrocarbures paraffiniques produits par traitement à l'hydrogène desdites charges sont exclusivement des paraffines linéaires (n-paraffines), c'est à dire non ramifiées. En conséquence, il est possible de les transformer :

- soit en sulfonates de paraffines longues par sulfonation,
- soit en hydrocarbures aromatiques alkylés (comme par exemple les "Linear Alkyl Benzenes") au moyen d'une réaction d'alkylation entre les paraffines linéaires et des hydrocarbures aromatiques choisi parmi le benzène, le toluène ou encore les xylènes.

Les composés tels que les sulfonates de paraffines longues ou les hydrocarbures aromatiques alkylés ont des propréiés intéressantes de tensio-actifs permettant leur utilisation en tant que bases pour produits détergents.

**État de l'art**

La demande de brevet US2007/0281875 propose un procédé de conversion des huiles et graisses naturelles comportant les étapes suivantes : a/ conversion par élimination de l'oxygène pour produire des paraffines et des oléfines par décarboxylation, en utilisant un catalyseur acide, comportant notamment une zéolithe ; b/ fonctionnalisation des paraffines et oléfines produites à l'étape a/ par réaction OXO afin de produire des alcools, ou par alkylation par des composés aromatiques de type benzène ou toluène au moyen d'un catalyseur d'alkylation ; et c/ sulfonation ou alkoxylation des composés de type alkyl-benzènes produits lors de l'étape précédente, afin de produire des composés entrant dans la composition des détergents.
La demande de brevet US2007/0281875 ne mentionne pas de contrôle strict de la température dans la première étape, l'élément critique de ladite première étape étant l'utilisation d'un catalyseur acide activé et en particulier l'utilisation d'une zéolithe.

La demanderesse a mis en évidence qu'une combinaison spécifique de conditions opératoires lors de la première étape d'hydrotraitement consistant à transformer en favorisant la voie décarboxylation, une charge issue de sources renouvelables en composés hydrocarbures constitués de paraffines linéaires, suivie d'une seconde étape de transformation des paraffines linéaires produites, permettait la production de composés tensioactifs qui, de par leurs propriétés intéressantes comme agents de solubilisation, ont des applications en particulier comme détergents, savons liquides, émulsifiants, dispersants.
En particulier, l'étape de transformation des paraffines linéaires produites lors de la première étape consiste avantageusement soit en a/ une réaction d'alkylation des paraffines linéaires avec des hydrocarbures aromatiques choisis parmi le benzène, le toluène, les xylènes ou des mélanges de tels hydrocarbures aromatiques en hydrocarbures aromatiques alkylés soit en ; b/ la sulfonation des paraffines linéaires, de façon à produire des sulfonates d'alcanes, lesdits hydrocarbures aromatiques alkylés et lesdits sulfonates d'alcanes étant des tensio-actifs.

**Objet de l'invention**

L'objectif de la présente invention est alors de proposer un procédé de production de composés tensioactifs à partir de charges d'origine renouvelable contenant des lipides, triglycé-rides et/ou acides gras et/ou esters.

Ainsi, la présente invention concerne un procédé de production de composés tensio-actifs à partir d'une charge issue de sources renouvelables comprenant au moins les étapes suivantes :

a) une étape d'hydrotraitement de ladite charge pour produire un effluent contenant au moins des composés hydrocarbures constitués de paraffines linéaires, en présence d'hydrogène en excès de la consommation d'hydrogène théorique et à des conditions d'hydrotraitement dans un réacteur à lit fixe ayant plusieurs zones catalytiques disposées en série et comprenant un catalyseur d'hydrotraitement, dans laquelle :

i) le flux de la charge totale F est divisé en un certain nombre de différents flux partiels F1 à Fn égal au nombre de zones catalytiques n dans le réacteur, le premier flux partiel F1 est injecté dans la première zone catalytique, le deuxième flux partiel F2 est injecté dans la deuxième zone catalytique et ainsi de suite, si n est supérieur à 2,
les différents flux partiels étant injectés dans les zones catalytiques successives dans des proportions croissantes de telle manière que F1/F soit inférieur à F2/F, lui-même inférieur a F3/F et ainsi de suite jusqu'à F(n-1)/F soit inférieur à Fn/F,
la température du flux injecté à l'entrée de la première zone catalytique Z1 comprenant le flux partiel de la charge F1 mélangé au flux gaz riche en hydrogène H1 entrant dans la zone Z1 et à un recycle liquide R, (F1 + R + H1) étant supérieure à 250 °C, et la température des flux à l'entrée des zones catalytiques suivantes Z2 à Zn étant supérieures à 300 °C,
ii) ledit effluent contenant au moins des composés hydrocarbures constitués de paraffines linéaires est soumis à au moins une étape de séparation permettant de séparer une fraction gazeuse contenant l'hydrogène, le CO, le $CO_2$, l'$H_2S$, l'eau et les gaz légers et une fraction liquide d'hydrocarbures constitués de paraffines linéaires,
iii) au moins une partie R de ladite fraction liquide d'hydrocarbures constitués de paraffines linéaires est recyclée à la première zone catalytique Z1 de telle sorte que le rapport massique entre le flux dudit recycle R et le flux partiel F1 introduit dans la première zone catalytique Z1, R/F1, soit supérieur ou égal à 8,

b) une étape de transformation d'au moins une partie de ladite fraction liquide d'hydrocarbures constitués de paraffines linéaires issue de l'étape a) en composés tensio-acifs.

L'introduction de la charge en proportions croissantes couplée avec un recycle important sur la première zone et des températures des flux à l'entrée des différentes zones catalytiques d'hydrotraitement spécifiques, permet d'atteindre, par un profil montant de températures, une zone suffisamment chaude en fin de zone catalytique pour favoriser les réactions de décarboxylation par rapport aux réactions d'hydrodésoxygénation. Par ailleurs, dans le cas où la charge initiale comporte des triglycérides et/ou des acides gras libres et/ou des esters présentant des chaines hydrocarbonées avec un nombre d'atomes de carbone supérieur à 17, l'étape a) du procédé selon la présente invention permet de maximiser la proportion d'hydrocarbures de type paraffines linéaires avec un nombre d'atomes de carbone d'au plus 17.

Par ailleurs, l'injection étagée de la charge, consistant à introduire dans les zones réactionnelles d'hydrotraitement des proportions croissantes de charge fraîche, permet de gérer le plus efficacement l'évolution des températures dans chaque zone catalytique.

Un autre avantage de l'invention réside dans le fait que les températures des flux à l'entrée des différentes zones catalytiques d'hydrotraitement sont telles que toute dégradation de ladite charge d'origine renouvelable par polymérisation ou craquage avant sa transformation est évité.

## Description détaillée de l'invention

Les charges issues de sources renouvelables traitées dans le procédé selon la présente invention sont choisies parmi les huiles et graisses d'origine végétale ou animale, ou des mélanges de telles charges, contenant des triglycérides et/ou des acides gras libres et/ou des esters.

Lesdites charges sont généralement caractérisées par une masse molaire élevée (supérieure à 800 g/mole le plus souvent), et les chaînes d'acides gras qui les composent ont un nombre d'atomes de carbone compris entre 4 et 24, et un nombre d'insaturations par chaîne généralement compris entre 0 et 3, avec des valeurs supérieures qui peuvent être atteintes sur certaines charges spécifiques. Parmi les charges qui peuvent être traitées dans le procédé selon la présente invention, on peut citer, cette liste n'étant pas exhaustive : les huiles végétales telles que les huiles de colza, de jatropha, de soja, de palme, de tournesol, d'olive, de coprah, de cameline, des huiles de poisson ou des huiles algales hétérotrophes ou autotrophes, ou encore des graisses animales telles que le suif de boeuf, ou encore des résidus issus de l'industrie papetière (tels que le "tall oil"), ou des mélanges de ces diverses charges. Les différents flux partiels de charge sont avantageusement de nature identique ou différente. Un avantage du procédé selon l'invention consiste dans sa grande flexibilité, selon l'origine de la charge. Des charges différant notablement entre elles, en particulier de par leurs différents degrés d'insaturation des chaines hydrocarbonées, peuvent être converties totalement en ce qui concerne l'élimination de l'oxygène, tout en produisant à l'issue de cette étape d'hydrotraitement a) à des hydrocarbures paraffiniques exclusivement linéaires.

Toutes ces charges contiennent des teneurs en oxygène élevées, ainsi que, à des teneurs très variables selon l'origine des charges, en composés soufrés, mais aussi en composés azotés, et en métaux comme le phosphore, le calcium, le magnésium, le potassium ou le sodium. La teneur en métaux peut aller jusqu'à 2500 ppm. Les teneurs en azote et en soufre sont généralement comprises entre 1 ppm et 100 ppm poids environ et de préférence inférieures à 100 ppm, selon leur nature. Elles peuvent atteindre jusqu'à 1% poids sur des charges particulières.

La charge traitée peut avantageusement être brute, ou peut aussi avoir subi un traitement de raffinage ou pré-raffinage, dont le but est d'abaisser la teneur en métaux. Cette étape de prétraitement peut avoir été réalisée au préalable, ou dans une section de prétraitement placé en amont du réacteur d'hydrotraitement. Cette étape optionnelle de prétraitement consiste avantageusement en un traitement thermique, associé à un passage sur des solides tels que les alumines ou les silice-alumines, ou encore un traitement à la vapeur d'eau, ou encore un traitement à l'acide comme par exemple l'acide phosphorique, ou encore un traitement par résine échangeuse d'ions, ou encore une association de plusieurs de ces étapes de prétraitement. D'une manière générale, le prétraitement peut inclure toutes méthodes (dégommage, déphosphatation...) connus de l'homme du métier traitant du raffinage de l'huile à des fins alimentaires.

Dans la suite, l'invention sera décrite en se référant aux figures pour faciliter la compréhension, les figures ne limitant pas le caractère général de l'invention.

## Hydrotraitement

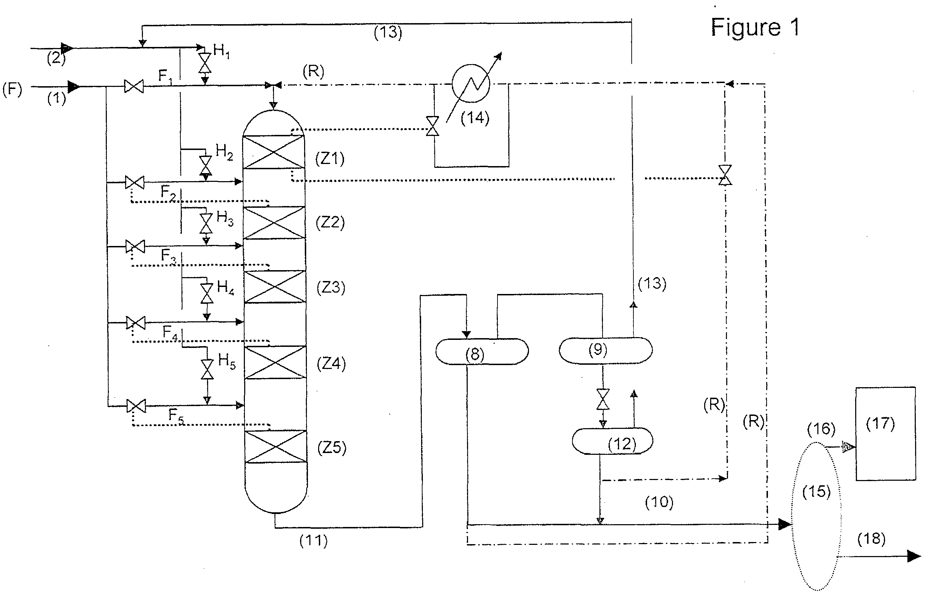

La charge encore appelée la charge fraîche, est injectée dans la ligne (1) représentée dans la figure 1. Cette charge est mélangée avec un gaz riche en hydrogène (2), mais qui peut également contenir d'autres composés hydrocarbures inertes, c'est à dire qui ne réagissent pas en tant que tels avec les constituants de la charge. L'hydrogène peut avantageusement venir d'un appoint d'hydrogène et/ou du recyclage du gaz riche en hydrogène issu de l'étape de séparation après l'étape d'hydrotraitement. En pratique, l'hydrogène d'appoint peut provenir du reformage à la vapeur ou du reformage catalytique, et sa pureté en hydrogène est le plus souvent comprise entre 75 et 95 % volume, les autres gaz présents étant généralement le méthane, l'éthane, le propane et le butane. Le gaz riche en hydrogène issu de l'étape de séparation après l'étape d'hydrotraitement subit de préférence préalablement un ou plusieurs traitements de purification intermédiaire avant d'être recyclé dans le procédé d'hydrotraitement.

Selon une caractéristique de l'invention, l'hydrogène utilisé est en excès par rapport à la consommation théorique, l'excédent d'hydrogène représente au moins 50% de cette consommation théorique, de préférence entre 75 et 400%, et de façon encore préférée entre 100% et 300%, 150% étant une valeur typique. La quantité d'hydrogène mise en jeu est contrôlée par la pression partielle d'hydrogène.

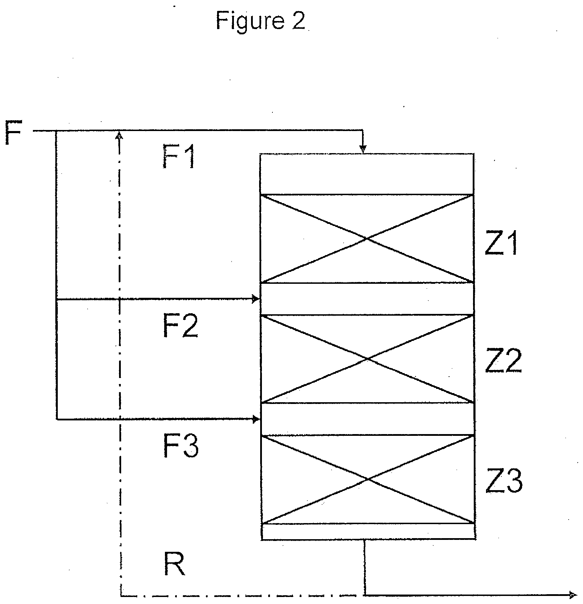

Pour une compréhension plus aisée de la présente invention, les définitions suivantes sont introduites. Elles font référence à la figure 2. Le réacteur comprend n zones catalytiques. Tous les flux sont exprimés en débit massique.

F: flux total de la charge renouvelable traitée dans le procédé

F1 : flux partiel de la charge introduit dans la première zone catalytique Z1

F2 : flux partiel de la charge introduit dans la deuxième zone catalytique Z2

F3 : flux partiel de la charge introduit dans la troisième zone catalytique Z3 et ainsi de suite...

Fn : flux partiel de la charge introduit dans la dernière zone catalytique Zn

R : flux de recycle, recyclée dans la première zone catalytique Z1

Le recycle total (RT) est défini comme le rapport massique entre le flux du recycle envoyé dans la première zone catalytique Z1 (R) et le flux total de la charge renouvelable (F) :

$$RT = R/F$$

Le recycle local (RF1) de la première zone catalytique, est défini comme le rapport massique entre le flux du recycle envoyé dans la première zone catalytique Z1 (R) et le flux partiel de la charge introduite dans la première zone catalytique 1 (F1) :

$$RF1 = R/F1$$

Excepté durant la phase de démarrage du procédé, l'agent diluant qui est recyclé au niveau de la zone catalytique Z1, donc en amont du premier lit catalytique, est constitué par une partie du produit hydrocarbure liquide et constitué de paraffines linéaires, sortant de la section hydrotraitement. Cet agent diluant recyclé à l'entrée de la première zone catalytique, est appelé aussi recycle liquide ou recycle dans la suite de cette description, et son débit est noté R dans les définitions qui précèdent. La section hydrotraitement du procédé étant conçue de façon à convertir totalement les charges traitées, le recycle liquide produit est un flux d'hydrocarbure exempt d'oxygène, ce qui signifie que sa teneur en oxygène est inférieure à la limite de détection analytique, et est essentiellement composé de paraffines. En conséquence, ce recycle liquide est inerte vis-à-vis des réactions d'hydrotraitement, et joue ainsi pleinement son rôle de diluant de la charge, ce qui permet de limiter l'élévation de température dans la première zone catalytique, due à l'exothermie des réactions qui s'y produisent. Néanmoins, l'objectif est, pour une capacité donnée, c'est à dire pour un débit massique donné de charge traitée, noté F, de limiter la quantité de recycle liquide injecté dans la première zone, noté R, ceci afin de limiter le débit total du flux alimentant cette zone catalytique. Ceci permet d'utiliser des réacteurs d'hydrotraitement de dimensions comparables à celles des réacteurs d'hydrotraitement de coupes pétrolières comme les gazoles (et donc de limiter les coûts), de limiter les pertes de charges et d'éviter des phénomènes d'engorgement du réacteur. En pratique, selon une caractéristique préférée de l'invention, le rapport massique entre le flux du recycle envoyé dans la première zone catalytique Z1 (noté R) et le flux total de la charge renouvelable traité (noté F), aussi appelée recycle total (RT) est de préférence inférieur à 1.0, et de manière encore préférée inférieur à 0.5, lesdits flux étant exprimés en débit massique.

Conformément à l'invention, les flux partiels de charge sont introduits dans les différentes zones catalytiques (F1 injecté dans la zone Z1, F2 dans la zone Z2, etc...) en faisant en sorte que des proportions croissantes de charge soient injectées dans les zones catalytiques successives. Ainsi, conformément à l'invention, F1/F est inférieur à F2/F, lui-même étant inférieur à F3/F, etc...,. Plus généralement F(n-1)/F est inférieur à Fn/F, pour le cas général où n est le nombre de zones catalytiques mises en jeu.

L'avantage apporté par une telle distribution de la charge dans les différentes zones catalytiques successives, réside dans le fait que les températures de sortie des différentes zones suivent un profil montant, ce qui permet d'atteindre en sortie de chaque zone catalytique des températures suffisantes pour éliminer complètement l'oxygène et produire ainsi un effluent contenant au moins des composés hydrocarbures constitués de paraffines linéaires.

Conformément à l'invention, la température du flux injecté à l'entrée de la première zone catalytique Z1, comprenant et de préférence constitué du, le flux partiel de la charge F1 mélangé au flux gaz riche en hydrogène H1 entrant dans la zone Z1 et au recycle liquide R constitué par une partie de la fraction liquide d'hydrocarbures constituée de paraffines linéaires, est supérieure à 250 °C, de préférence supérieure à 260 °C et de manière préférée supérieure à 270 °C et la température des flux à l'entrée des zones catalytiques suivantes Z2 à Zn est supérieure à 300 °C, de préférence supérieure à 310 °C et de manière préférée supérieure à 320 °C.

Le flux à l'entrée des zones catalytiques suivants la première zone, et en particulier, le flux à l'entrée de la deuxième zone catalytique comprend :

- le flux partiel de la charge introduit dans la deuxième zone catalytique Z2 (F2), telle que le rapport massique F2/F soit supérieur au rapport massique F1/F,
- le recycle liquide injecté en entrée de zone Z1, composé exclusivement d'hydrocarbures paraffiniques linéaire et ayant traversé la zone Z1,
- le flux de gaz riche en hydrogène H2 entrant dans Z2,
- l'effluent formé par la conversion de la charge dans la zone Z1, correspondant au débit F1. Les hydrocarbures liquides présents dans cet effluent sont exempts d'oxygène et exclusivement des hydrocarbures paraffiniques linéaires.

Et ainsi de suite pour toutes les zones catalytiques suivant la première.

La température à la sortie d'au moins une zone catalytique est de préférence supérieure à 320 °C, de manière préférée supérieure à 350 °C. Les températures à la sortie de chacune des zones catalytiques doivent être de préférence inférieures à 400 °C, de façon à limiter la désactivation du catalyseur par cokage.

Conformément à l'invention, un flux partiel de charge F1 est injecté dans la première zone catalytique de telle sorte que le rapport massique entre le flux du recycle liquide (R) injecté en entrée de la première zone catalytique Z1, et le flux partiel de charge injectée en entrée de zone 1 (F1), soit supérieur ou égal à 8 et de préférence supérieur ou égal à 10, lesdits flux étant exprimés en débit massique. Ce rapport est aussi appelé recycle local.

L'utilisation d'un tel agencement des flux de charge et de recycle liquide combiné à des températures d'entrée de flux dans les différentes zones catalytiques spécifiques permet :

- d'une part, d'obtenir une température homogène dans toute la section du réacteur en sortie de zone Z1.
- d'autre part, d'atteindre en sortie de zone Z1 une température suffisante, permettant d'amorcer les réactions de décarboxylation et ainsi de maximiser la proportion de paraffines linéaires ayant un nombre d'atomes de carbones d'au plus 17 dans l'hydrocarbure liquide produit en sortie de la zone Z1, dans le cas où la charge initiale comprend des composés ayant un nombre d'hydrocarbures supérieurs à 17,
- d'atteindre des températures supérieures en sortie des zones catalytiques suivant la zone Z1 (zones Z2 à Zn), et suffisantes pour augmenter l'importance relative des réactions de décarboxylation par rapport aux réactions de désoxygénation.

En effet, l'introduction de la charge en proportions croissantes couplée avec un recycle important sur la première zone et des températures des flux à l'entrée des différentes zones catalytiques d'hydrotraitement spécifiques, permet d'atteindre, par un profil montant de températures, une zone suffisamment chaude en fin de zone catalytique pour favoriser les réactions de décarboxylation par rapport aux réactions d'hydrodésoxygénation.

Le ratio du recycle local supérieur ou égal à 8 signifie qu'on injecte relativement peu de charge sur la première zone, permettant ainsi d'injecter le restant de la charge dans des proportions plus importantes et croissantes dans les zones catalytiques successives. L'augmentation de la quantité de la charge injectée dans les zones successives permet d'obtenir un profil montant en températures d'entrée et de sortie des différentes zones.

Durant les phases de démarrage, une large gamme d'hydrocarbures peut être injectée, jusqu'à ce qu'une quantité suffisante de produit paraffinique soit disponible pour être recyclée en entrée de zone Z1.

La charge est alimentée par la ligne (1), comme le montre la figure 1, tandis que le gaz riche en hydrogène l'est par la ligne (2). La charge est distribuée en différents flux F1, F2,..., Fn alimentant les différentes zones catalytiques successives. Le gaz riche en hydrogène est distribué en autant de flux H1, H2, ..., Hn. Le flux de charge F1 est mélangée au flux de gaz (H1), le flux de charge (F2) est mélangé au flux de gaz (H2), et ainsi de suite jusqu'à la n-ième zone catalytique.

La température des flux de charge F1, F2,..., Fn est inférieure à 150 °C, de préférence inférieure à 100 °C, et de manière encore préférée inférieure à 80 °C. Elle doit être suffisante pour permettre un abaissement de viscosité suffisant et donc un transfert adéquat des bacs de stockage jusqu'à la section réactionnelle de l'hydrotraitement. Il n'est ni utile ni souhaitable de porter la température de la charge à des valeurs supérieures en l'absence d'hydrogène, de façon à éviter toute dégradation des charges par suite de polymérisation et par suite de cokage par exemple.

La température du gaz riche en hydrogène, qui est mélangé à la charge peut être ajustée afin de contribuer à obtenir la température désirée des flux en entrée des différentes zones catalytiques.

En pratique, comme une élévation de température se produit lors de la compression du gaz riche en hydrogène, l'hydrogène est éventuellement refroidi après compression. Le plus souvent, la température du gaz riche en hydrogène est comprise entre 40 et 100 °C, par exemple 50 °C.

L'introduction de la charge en proportions croissantes couplée avec un recycle important sur la première zone et des températures des flux à l'entrée des différentes zones catalytiques d'hydrotraitement spécifiques, doivent être soigneusement réglées de façon à permettre l'amorçage de l'ensemble des réactions et en particulier des réactions de décarboxylation/décarbonylation conduisant à la formation de $CO_2$ et CO. Les températures des flux à l'entrée des différentes zones catalytiques peuvent être ajustées selon la nature de la charge. Le volume de catalyseur mis en jeu dans cette zone catalytique est adapté de façon à ce que la conversion -c'est à dire le taux d'élimination de l'oxygène- soit complète en sortie de cette zone Z1.

En sortie de la zone catalytique Z1, est ajouté le second flux de charge F2, qui représente une plus grande proportion de charge que celle injectée en entrée de zone Z1. Cette charge injectée en entrée de zone Z2, peut être strictement identique à celle injectée en entrée de zone Z1, mais aussi être une charge d'origine renouvelable mais de nature différente. Ce flux de charge est additionné au flux de gaz riche en hydrogène ($H_2$), le tout est injecté dans la zone réactionnelle, où il est mélangé à l'effluent provenant de la zone Z1. Ceci permet un abaissement de la température du produit formé à l'issue de la zone Z1, et la température en entrée de la zone Z2 est donc généralement supérieure à celle en entrée de zone Z1 et doit être ajustée à une température supérieure à 300 °C selon l'invention. Les mêmes familles de réactions se produisent dans la zone Z2 et la zone Z1, avec une cinétique un peu plus rapide dans la zone Z2 en raison d'une température moyenne supérieure.

Le même principe se déroule ensuite dans les zones catalytiques successives, le flux de charge étant additionné au produit totalement converti formé dans les zones catalytiques antérieures.

Au fur et à mesure que la charge se transforme en hydrocarbures paraffiniques linéaires dans une zone catalytique, la température augmente dans la zone, les réactions d'hydrogénation et de décarboxylation étant des réactions fortement exothermiques.

Les ratios entre débits d'hydrogène ajoutés à chacun de ces flux (F1), ... (Fn) et débits massiques de charge (F1), ... (Fn), sont du même ordre de grandeur pour l'ensemble des zones catalytiques, le ratio entre le débit d'hydrogène et le débit de charge est compris entre 300 et 1500 $Nm^3/m^3$, de préférence entre 600 et 900 $Nm^3/m^3$.

Optionnellement, il est possible d'injecter entre les zones catalytiques, un flux liquide complémentaire si le besoin de diluer davantage la charge se fait sentir.

Selon une variante préférée, des vannes de régulation des flux partiels de charge et d'hydrogène peuvent être contrôlées par les valeurs de température au niveau des entrées et des sorties des zones catalytiques de manière à ajuster les flux partiels de charge et d'hydrogène ainsi que le flux du recycle liquide pendant le fonctionnement. De cette manière la température désirée à l'entrée des zones catalytiques et dans les zones catalytiques est maintenue. Ceci est illustré par les lignes pointillées dans la figure 1. De même, le contrôle de la température peut être réalisé en variant la température de la charge et/ou de l'hydrogène injectées et/ou du recycle (via l'échangeur (14)) dans le système de réacteur (voir ci-dessus).

Le réacteur d'hydrotraitement de l'étape a) du procédé selon l'invention peut contenir un nombre variable de zones catalytiques. Il comprend habituellement entre 3 et 10 zones catalytiques, de préférence entre 3 et 6 zones catalytiques. On entend par zone catalytique un lit catalytique. Chaque zone catalytique peut comprendre une ou plusieurs couches de catalyseurs, identiques ou différentes, éventuellement complétée par des couches inertes. Les zones catalytiques peuvent contenir des catalyseurs identiques ou différents.

Conformément à l'invention, l'étape a) d'hydrotraitement opère en présence d'un catalyseur d'hydrotraitement. Le type de catalyseurs utilisés dans l'étape a) hydrotraitement de ce procédé, est bien connu dans l'art antérieur.

De préférence, le catalyseur utilisé dans l'étape a) d'hydrotraitement ne comprend pas de zéolithe.

Les catalyseurs utilisés dans la section hydrotraitement du procédé selon l'invention, peut être une association des catalyseurs décrits dans ce qui suit.

Le catalyseur utilisé dans ladite étape a) peut avantageusement être sous forme supporté ou massique.

Le catalyseur utilisé dans ladite étape a) peut avantageusement être sous forme métallique ou sulfure.

Dans le cas où ledit catalyseur est un catalyseur supporté, ledit catalyseur d'hydrotraitement comprend avantageusement un ou plusieurs éléments des groupes 6, 8, 9 et 10 de la classification périodique, de préférence choisi parmi le nickel, le molybdène, le tungstène et/ou le cobalt, pris seuls ou en mélange et un support choisi dans le groupe formé par l'alumine, la silice, les silices-alumines, la magnésie, les argiles et les mélanges d'au moins deux de ces minéraux. Ce support peut également avantageusement renfermer d'autres composés et par exemple des oxydes choisis dans le groupe formé par l'oxyde de bore, la zircone, l'oxyde de titane, l'anhydride phosphorique. Le support préféré est un support d'alumine et de manière très préférée de l'alumine $\eta$, $\delta$ ou $\gamma$.

La teneur en oxydes de métaux des groupes 8 est avantageusement comprise entre 0,5 et 10 % en poids d'oxyde et de préférence entre 1 et 5 % en poids d'oxyde et la teneur en oxydes de métaux des groupes 6 est avantageusement comprise entre 1 et 30 % en poids d'oxyde, de préférence de 5 à 25 % en poids, les pourcentages étant exprimés en % poids par rapport à la masse totale du catalyseur.

La teneur totale en oxydes de métaux des groupes 6 et 8 dans le catalyseur utilisé est avantageusement comprise entre 5 et 40 % en poids et de manière préférée comprise entre 6 et 30 % en poids par rapport à la masse totale du catalyseur.

Le rapport pondéral exprimé en oxyde métallique entre métal (ou métaux) du groupe 6 sur métal (ou métaux) du groupe 8 est avantageusement compris entre 20 et 1 et de manière préférée entre 10 et 2.

Ledit catalyseur utilisé dans l'étape d'hydrotraitement du procédé selon l'invention peut également avantageusement contenir un élément dopant choisi parmi le phosphore et le bore, pris seuls ou en mélange. Ledit élément dopant peut être introduit dans la matrice ou de préférence être déposé sur le support. On peut également déposer du silicium sur le support, seul ou avec le phosphore et/ou le bore et/ou le fluor.

La teneur en poids d'oxyde dudit élément dopant est avantageusement inférieure à 20 % et de manière préférée inférieure à 10 % et elle est avantageusement d'au moins 0.001 % par rapport à la masse totale du catalyseur.

Dans le cas où ledit catalyseur est sous forme sulfure, un composé soufré tel que le Di-Méthyl-Di-Sulfure (DMDS)est avantageusement ajouté à l'ensemble des flux de charge. Dans les conditions de température l'étape a) d'hydrotraitement, ledit composé se décompose en $H_2S$ et en méthane. Ce dispositif permet de conserver sous leur forme sulfure les catalyseurs d'hydrotraitement utilisés dans le présent procédé, et ainsi de maintenir une activité catalytique suffisante tout au long du cycle. Les teneurs en DMDS injectées préconisées sont comprises entre 10 et 50 ppm poids équivalent soufre par rapport à la charge. En pratique, une addition de DMDS correspondant à 50 ppm poids équivalent soufre par rapport à la charge, est suffisante pour conserver l'activité catalytique tout au long du cycle.

Dans le cas où ledit catalyseur est sous forme métallique, ledit catalyseur comprend avantageusement un métal choisi parmi le nickel, le platine, le palladium, le ruthénium et la rhodium, supportés sur un support choisi parmi l'alumine, la silice, la silice-alumine, le carbone, le charbon actif, la cérine et la zircone ou d'un mélange de ces composés.

Un catalyseur métallique préféré comprend de 0,05 à 10% en poids et de préférence de 0,1 et 5% poids d'au moins un métal noble du groupe VIII, de préférence choisis parmi le platine et le palladium et de manière préférée, ledit métal noble étant le platine, déposé sur un support.

Un autre catalyseur métallique préféré comprend de 5 à 75 % poids d'un métal non noble d'u groupe VIII et de préférence de Nickel, déposé sur un support.

On ne sortirait pas du cadre de la présente invention en utilisant dans l'étape a) d'hydrotraitement du procédé selon l'invention, de manière simultanée ou de manière successive, un seul catalyseur ou plusieurs catalyseurs différents dans les zones catalytiques.

L'étape a) d'hydrotraitement du procédé selon l'invention permet donc, par la combinaison de conditions mises en oeuvre et explicitées ci-dessus, de favoriser la désoxygénation selon la voie décarboxylation/décarbonylation. L'effluent contenant au moins des composés hydrocarbures constitués de paraffines linéaires issu de l'étape a) comporte donc majoritairement des composés hydrocarbonés paraffiniques linéaires impairs par rapport aux composés hydrocarbures pairs.

La sélectivité pour la voie décarboxylation/décarbonylation est mise en évidence par la mesure du rendement total en hydrocarbure ayant un nombre d'atomes de carbone impair et du rendement total en hydrocarbure ayant un nombre d'atome de carbone pair dans la fraction liquide d'hydrocarbures paraffiniques linéaires. Les rendements en hydrocarbures impairs et pairs permettant d'accéder à la sélectivité de réaction (décarbonylation/décarboxylation/HDO) sont obtenus par analyse chromatographique en phase gazeuse des effluents liquides issu de l'étape a). La technique de mesure par analyse chromatographique en phase gazeuse est une méthode connue de l'homme du métier.

Sauf indication contraire, le procédé selon l'invention est opéré dans des conditions d'hydrotraitement généralement connues dans l'art antérieur, comme par exemple dans le brevet EP 1 741 768. La pression totale est généralement comprise entre 20 et 150 bar (2 MPa et 15 MPa), et de préférence entre 50 et 100 bar (5 MPa et 10 MPa).

Comme indiqué précédemment, l'hydrogène est utilisé en excès. Dans le procédé selon l'invention, le ratio entre le débit d'hydrogène et le débit de charge brute est compris entre 300 et 1500 $Nm^3/m^3$, de préférence entre 600 et 900 $Nm^3/m^3$.

Une opération satisfaisante du procédé selon l'invention conduit à utiliser une VVH globale (définie comme étant le ratio entre le débit volumique total de charge brute traitée et le volume total de catalyseur dans la section hydrotraitement) comprise entre 0.1 et 5.0 $h^{-1}$, de préférence entre 0.1 et 1.5 $h^{-1}$.

Les températures utilisées dans les différentes zones de la section hydrotraitement, doivent être soigneusement contrôlées, afin d'éviter le plus possible les réactions indésirables telles que les réactions de polymérisation de la charge, conduisant au dépôt de coke et donc à la désactivation du catalyseur, tout en réalisant la conversion totale de la charge, c'est à dire en éliminant totalement les composés oxygénés, de préférence par décarboxylation/décarbonylation. D'une manière générale, le procédé selon l'invention opère à une température comprise entre 200 et 400 °C.

Le procédé selon l'invention utilise des réacteurs en lit fixe à écoulement ruisselant connus de l'homme du métier. Les réactifs (charge et hydrogène) sont introduits dans le réacteur suivant un écoulement descendant en co-courant du haut vers le bas du réacteur. De tels réacteurs sont par exemple décrits dans le document US 7 070 745.

Entre chaque zone catalytique, il est possible d'injecter de l'hydrogène supplémentaire, afin de profiter d'un effet de trempe (quench selon le terme anglo-saxon) et d'atteindre les températures souhaitées en entrée de la zone catalytique suivante. Ainsi, des boîtes de quench sont possiblement installées entre chaque zone catalytique, afin d'assurer une meilleure homogénéité des températures sur toute la section du réacteur, et pour l'ensemble des zones catalytiques.

De la même manière, des distributeurs sont possiblement installés entre chaque zone catalytique, afin de garantir une alimentation homogène en charge liquide, sur toute la section du réacteur, et pour l'ensemble des zones catalytiques.

Un avantage du procédé selon l'invention consiste dans sa grande flexibilité, selon l'origine de la charge. Des charges différant notablement entre elles, en particulier de par leurs différents degrés d'insaturation des chaines hydrocarbonées, peuvent être converties totalement en ce qui concerne l'élimination de l'oxygène (ce qui amène une efficacité maximale de dilution de la charge brute dans la zone suivante).

**Séparation**

Conformément à l'invention, l'effluent contenant au moins des composés hydrocarbures constitués de paraffines linéaires formé dans la dernière zone catalytique issu de l'étape a) est soutiré dans la ligne (11), et est soumis ensuite à au moins une étape de séparation permettant de séparer une fraction gazeuse contenant l'hydrogène, le CO, le $CO_2$, l'$H_2S$, l'eau et les gaz légers et une fraction liquide d'hydrocarbures constitués de paraffines linéaires.

Selon une variante, la séparation peut être effectuée en une seule étape par un séparateur haute température haute pression (8) opérant sans réduction de pression à une température comprise entre 145 °C et 280 °C.

Selon une autre variante, l'étape de séparation comprend une séparation en deux étapes sans réduction de pression, la première séparation étant effectuée entre 145 °C et 280 °C dans un séparateur à haute température (8), la deuxième étant effectuée entre 25 °C et 100 °C dans un séparateur à basse température (9). Dans un mode de réalisation préféré, le condensat de la fraction obtenu à partir de la deuxième étape de séparation est introduit dans un récipient de dégazage (12).

De préférence, l'effluent liquide issu de la séparation gaz/liquide précédente subit ensuite une séparation (non représentée sur la figure) d'au moins une partie et de préférence la totalité restante de l'eau formée, d'au moins une base hydrocarbonée liquide, l'eau étant produite lors des réactions d'hydrodésoxygénation.
Le but de cette étape est de séparer l'eau de l'effluent hydrocarboné liquide. On entend par élimination de l'eau, l'élimination de l'eau produite par les réactions d'hydrodésoxygénation (HDO).
L'élimination de l'eau peut être réalisée par toutes les méthodes et techniques connues de l'homme du métier, telles que par exemple par séchage, passage sur un dessicant, flash, extraction par solvant, distillation et décantation ou par association d'au moins deux de ces méthodes.

De façon optionnelle, une étape de purification finale des différents polluants peut être mise en oeuvre par des méthodes connues de l'homme du métier telles que par exemple par strippage à la vapeur ou à l'azote ou par coalescence et/ou masse de captation.

Conformément à l'invention, au moins une partie de la fraction liquide d'hydrocarbures constitués de paraffines linéaires issue de l'étape de séparation est recyclée à la première zone catalytique Z1 de telle sorte que le rapport massique entre le flux dudit recycle et le flux partiel F1 introduit dans la première zone catalytique Z1 soit supérieur ou égal à 8 et de préférence supérieure ou égale à 10.

La partie de la fraction liquide d'hydrocarbures constitués de paraffines linéaires issue de l'étape de séparation (10) qui n'est pas recyclée pour être additionnée au flux de charge injectée dans la zone Z1 comme recycle liquide (R), peut avantageusement être envoyée dans une section optionnelle de fractionnement (15), pour séparer une fraction liquide d'hydrocarbures via la conduite (16) constitués de paraffines linéaires comportant au plus 17 atomes de carbone (fraction C17-), et une fraction liquide d'hydrocarbures constitués de paraffines linéaires comportant 18 atomes de carbone ou plus (C18+) via la conduite (18).
Dans une variante, seule les paraffines linéaires comportant au plus 17 atomes de carbone sont envoyées dans l'étape b) de transformation (zone (16)) du procédé selon l'invention.

Le gaz contenant l'hydrogène (13) qui a été séparé lors de l'étape de séparation ii) du procédé selon l'invention est, si nécessaire, avantageusement au moins en partie traité pour réduire sa teneur en légers ($C_1$ à $C_4$). De même, il subit avantageusement un ou plusieurs traitements de purification intermédiaire, de préférence au moins un lavage avec au moins une amine, suivi de préférence d'une méthanation et/ou d'une séparation par adsorption modulée en pression (en anglais Pressure Swing Adsorption ou (PSA)), avant d'être recyclé.
On peut avantageusement introduire l'hydrogène de recycle, de préférence purifié, soit avec la charge entrant dans l'étape a) d'hydrotraitement selon l'invention, soit sous forme d'hydrogène de trempe entre les lits de catalyseurs d'hydrotraitement selon l'invention.

**Transformation en composés tensio-actifs**

Conformément à l'invention, au moins une partie de ladite fraction liquide d'hydrocarbures constitués de paraffines linéaires (10) issue de l'étape a) et de préférence une fraction liquide d'hydrocarbures constitués de paraffines linéaires comportant au plus 17 atomes de carbone (fraction C17-), est envoyée dans une étape b) de transformation en composés tensio-acifs.
L'étape b) de transformation en composés tensio-acifs a lieu dans une zone (15).

De préférence, ladite étape b) est :

- soit une étape d'alkylation d'au moins une partie de ladite fraction liquide d'hydrocarbures constitués de paraffines linéaires issue de l'étape a) et de préférence d'une fraction liquide d'hydrocarbures constitués de paraffines linéaires comportant au plus 17 atomes de carbone (fraction C17-), par des hydrocarbures aromatiques choisis parmi le benzène, le toluène, les xylènes ou des mélanges de ces hydrocarbures, pour produire des composés aromatiques alkylés par des chaînes paraffiniques longues,

- soit une étape de sulfonation d'au moins une partie de ladite fraction liquide d'hydrocarbures constitués de paraffines linéaires issue de l'étape a) et de préférence d'une fraction liquide d'hydrocarbures constitués de paraffines linéaires comportant au plus 17 atomes de carbone (fraction C17-), pour produire des composés sulfonates paraffiniques.

Les sulfonates paraffiniques, tout comme les aromatiques alkylés par des chaînes paraffiniques longues, ont des propriétés de tensio-actifs qui permettent leur utilisation comme bases détergents.

**Alkylation**

Dans le cas où ladite étape b) est une étape d'alkylation, ladite étape d'alkylation est réalisée en présence d'un catalyseur d'alkylation choisi parmi le chlorure d'aluminium, l'acide fluorhydrique seul ou en combinaison avec des zéolithes. Les catalyseurs d'alkylation sont des catalyseurs connus de l'état de l'art. Dans ladite étape d'alkylation, au moins une partie de ladite fraction liquide d'hydrocarbures constitués de paraffines linéaires issue de l'étape a) et de préférence une fraction liquide d'hydrocarbures constitués de paraffines linéaires comportant au plus 17 atomes de carbone (fraction C17-) est mise en contact avec des hydrocarbures aromatiques choisis parmi le benzène, le toluène, les xylènes ou des mélanges de ces composés.

La réaction d'alkylation peut être réalisée par voie catalyse homogène ou par voie catalyse hétérogène, comme indiqué dans la demande US2011275871 A. En amont de cette étape d'alkylation, au moins une partie de ladite fraction liquide d'hydrocarbures constitués de paraffines linéaires issue de l'étape a) et de préférence une fraction liquide d'hydrocarbures constitués de paraffines linéaires comportant au plus 17 atomes de carbone (fraction C17-) est de préférence soumise à une étape de déshydrogénation de manière à produire des composés mono-oléfiniques linéaires. Cette étape est particulièrement importante.

Les principales spécificités de la réaction de déshydrogénation résident dans le fait que l'équilibre thermodynamique limite le taux de conversion par passe et que la réaction est fortement endothermique. Ainsi, l'étape de déshydrogénation des n-paraffines se réalise de préférence à des températures supérieures à 400 °C avec un taux de conversion par passe compris entre 10 % et 25 %, limité par la thermodynamique. De préférence, l'étape de déshydrogénation opère sous hydrogène avec un rapport molaire H2/charge compris entre 0,5 et 10, la température étant comprise entre 400 et 800 °C, la pression totale étant comprise entre 0,01 et 2 MPa, la vitesse spatiale horaire étant comprise entre 0,5 et 300 h-1. Les catalyseurs de déshydrogénation des n-paraffines connus de l'homme de l'art sont multimétalliques : ils contiennent généralement du platine, de l'étain et de l'indium. La présence d'acidité sur le support favorise les réactions indésirables de craquage et d'isomérisation, produits non désirés..

L'effluent issu de l'étape optionnelle de deshydrogénation est ensuite mis en contact avec des hydrocarbures aromatiques choisis parmi le benzène, le toluène, les xylènes ou des mélanges de ces composés.

Dans le cas où ladite réaction d'alkylation est mise en oeuvre par catalyse homogène, on utilise un catalyseur liquide acide comme l'acide fluorhydrique HF ou l'acide sulfurique H$_2$SO$_4$. La température est comprise entre 10 et 80°C, de préférence entre 20 et 60 °C, et de manière encore préférée entre 30 et 50 °C. La pression est comprise entre 0.2 et 4 MPa, de manière préférée entre 0.2 et 3 MPa.. Le ratio volumique entre l'acide et le flux hydrocarbure olefine +paraffinique à convertir est compris entre 0.1 et 10 vol/vol, de préférence entre 1 et 3 vol/vol. Le ratio molaire entre le flux hydrocarbures aromatiques et le flux hydrocarbure paraffinique est compris entre 1 et 15 mol/mol, de préférence entre 5 et 12 mol/mol. Le temps de contact est compris entre 5 et 50 mn, de préférence entre 20 et 40 mn.

Dans le cas où ladite réaction d'alkylation est mise en oeuvre par catalyse hétérogène, on utilise un catalyseur solide acide amorphe ou zéolithique., choisi parmi la silice-alumine, la silice-alumine fluorée, les zéolithes de type FAU, MOR, MTW, OFF, MAZ, BEA ou EUO, ou les mélanges de ces solides. Les zéolithes contiennent un élément X choisi parmi Si ou Ge, et un élément T choisi parmi Al, Fe, Ga ou B.
La température est comprise entre 30 et 400 °C, de préférence entre 50 et 300 °C, et demanière encore préférée entre 70 et 300 °C. La pression est comprise entre 0.1 et 10 MPa, et de préférence entre 1 et 7 MPa. La vitesse volumique horaire ou VVH est comprise entre 0.01 et 200 h-1, de préférence entre 0.5 et 80 h-1. La mise en oeuvre peut être réalisée en lit fixe ou en lit mobile. Le ratio molaire entre le flux d'hydrocarbures aromatiques et le flux hydrocarures paraffiniques+olefinques est compris entre 2 et 50 mol/mol, de préférence entre 5 et 35 mol/mol.

Les composés tensio-actifs formés à l'issue de cette étape d'alkylation sont des hydrocarbures aromatiques alkylés par des paraffines linéaires longues, comme par exemple les alkylbenzenes (Linear Alkyl Benzenes).

**Sulfonation**

Dans le cas où ladite étape b) est une étape de sulfonation, au moins une partie de ladite fraction liquide d'hydrocarbures constitués de paraffines linéaires issue de l'étape a) et de préférence une fraction liquide d'hydrocarbures constitués de paraffines linéaires comportant au plus 17 atomes de carbone (fraction C17-) subit un traitement avec un acide choisi parmi l'acide sulfurique, l'acide chlorosulfonique, un oléum, du trioxyde de soufre SO$_3$, et de l'anhydride sulfureux, éventuellement en présence d'air. La chaleur dégagée lors de cette réaction exothermique de sulfonation, est éliminée par refroidissement extérieur, la température du mélange réactionnel étant avantageusement maintenue entre 20 et 100 °C.
La sulfonation est connue de l'homme du métier.
Une neutralisation est ensuite avantageusement réalisée au moyen d'un sel contenant les éléments parmi le sodium, le potassium, l'ammonium, le magnésium et les mélanges de ces sels. On obtient ainsi des composés de type sulfonates de paraffines longues (encore appelés Linear Alkyl Sulfonates ou LAS dans la terminologie anglo-saxonne), de formule générale $R\text{-}SO_3\text{-}X$, où R est une chaîne hydrocarbonée, et X est un cation pouvant être le sodium, le potassium, l'ammonium ou le magnésium. Ces composés sulfonates de paraffines longues ont des propriétés tensio-actives qui rendent leur utilisation intéressante pour différentes applications (détergents, émulsifiants, agents de solubilisation, agents moussants ou agents dispersants par exemple).

## Revendications

1. Procédé de production de composés tensio-actifs à partir d'une charge issue de sources renouvelables comprenant au moins les étapes suivantes :

a) une étape d'hydrotraitement de ladite charge pour produire un effluent contenant au moins des composés hydrocarbures constitués de paraffines linéaires, en présence d'hydrogène en excès de la consommation d'hydrogène théorique et à des conditions d'hydrotraitement dans un réacteur à lit fixe ayant plusieurs zones catalytiques disposées en série et comprenant un catalyseur d'hydrotraitement, dans laquelle :

i) le flux de la charge totale F est divisé en un certain nombre de différents flux partiels F1 à Fn égal au nombre de zones catalytiques n dans le réacteur, le premier flux partiel F1 est injecté dans la première zone catalytique, le deuxième flux partiel F2 est injecté dans la deuxième zone catalytique et ainsi de suite, si n est supérieur à 2, les différents flux partiels étant injectés dans les zones catalytiques successives dans des proportions croissantes de telle manière que F1/F soit inférieur à F2/F, lui-même inférieur à F3/F et ainsi de suite jusqu'à F(n-1)/F soit inférieur à Fn/F, la température du flux injecté à l'entrée de la première zone catalytique Z1 comprenant le flux partiel de la charge F1 mélangé au flux gaz riche en hydrogène H1 entrant dans la zone Z1 et à un recycle liquide R, (F1 + R + H1) étant supérieure à 250°C, et la température des flux à l'entrée des zones catalytiques suivantes Z2 à Zn étant supérieures à 300°C, ii) ledit effluent contenant au moins des composés hydrocarbures constitués de paraffines linéaires est soumis à au moins une étape de séparation permettant de séparer une fraction gazeuse contenant l'hydrogène, le CO, le CO2, l'H2S, l'eau et les gaz légers et une fraction liquide d'hydrocarbures constitués de paraffines linéaires, iii) au moins une partie R de ladite fraction liquide d'hydrocarbures constitués de paraffines linéaires est recyclée à la première zone catalytique Z1 de telle sorte que le rapport massique entre le flux dudit recycle R et le flux partiel F1 introduit dans la première zone catalytique Z1, R/F1, soit supérieur ou égal à 8,

b) une étape de transformation d'au moins une partie de ladite fraction liquide d'hydrocarbures constitués de paraffines linéaires issue de l'étape a) en composés tensio-acifs.

2. Procédé selon la revendication 1 dans lequel l'excédent d'hydrogène utilisé dans l'étape d'hydrotraitement est d'au moins 50% de la consommation théorique.

3. Procédé selon l'une des revendications 1 ou 2 dans lequel le rapport massique entre le flux du recycle envoyé dans la première zone catalytique et le flux total de la charge est inférieur à 1, de préférence inférieur à 0,5.

4. Procédé selon l'une des revendications 1 à 3 dans lequel la charge issue de sources renouvelables est choisie parmi les huiles et graisses d'origine végétale ou animale, ou des mélanges de telles charges, contenant des triglycérides et/ou des acides gras libres et/ou des esters.

5. Procédé selon l'une des revendications 1 à 4 dans lequel la température du flux injecté à l'entrée de la première zone catalytique Z1, c'est-à-dire de la charge mélangé au recycle liquide constitué par une partie de la fraction liquide d'hydrocarbures constituée de paraffines linéaires est supérieure à 260°C.

6. Procédé selon la revendication 5 dans lequel la température du flux injecté à l'entrée de la première zone catalytique Z1, c'est-à-dire de la charge mélangé au recycle liquide constitué par une partie de la fraction liquide d'hydrocarbures constituée de paraffines linéaires est supérieure à 270°C.

7. Procédé selon l'une des revendications 1 à 6 dans lequel la température des flux à l'entrée des zones catalytiques Z2 à Zn est supérieure à 310°C.

8. Procédé selon l'une des revendications 1 à 7 dans lequel l'hydrotraitement est opéré à une température comprise entre 200 et 400°C, à une pression totale comprise entre 2 MPa et 15 MPa, à une vitesse spatiale horaire comprise entre 0,1 h-1 et 5 h-1 et en présence d'une quantité totale d'hydrogène mélangée à la charge telle que le ratio hydrogène/charge soit compris entre 300 et 1500 Nm3 d'hydrogène/m3 de charge.

9. Procédé selon l'une des revendications 1 à 8 dans lequel le catalyseur d'hydrotraitement est un catalyseur sulfuré qui comprend un ou plusieurs éléments des groupes 6, 8, 9 et 10 de la classification périodique, de préférence choisi parmi le nickel, le molybdène, le tungstène et le cobalt, pris seuls ou en mélange et un support choisi dans le groupe formé par l'alumine, la silice, les silices-alumines, la magnésie, les argiles et les mélanges d'au moins deux de ces minéraux.

10. Procédé selon l'une des revendications 1 à 8 dans lequel le catalyseur d'hydrotraitement est un catalyseur métallique qui comprend un métal choisi parmi le nickel, le platine, le palladium, le ruthénium et la rhodium, supportés sur un support choisi parmi l'alumine, la silice, la silice-alumine, le carbone, le charbon actif, la cérine et la zircone ou d'un mélange de ces composés.

11. Procédé selon l'une des revendications 1 à 10 dans lequel au moins une partie de la fraction liquide d'hydrocarbures constitués de paraffines linéaires issue de l'étape de séparation est recyclée à la première zone catalytique Z1 de telle sorte que le rapport massique entre le flux dudit recycle et le flux partiel F1 introduit dans la première zone catalytique Z1 soit supérieur ou égal à 10.

12. Procédé selon l'une des revendications 1 à 11 dans lequel la partie de la fraction liquide d'hydrocarbures constitués de paraffines linéaires issue de l'étape de séparation qui n'est pas recyclée pour être additionnée au flux de charge injectée dans la zone Z1 comme recycle liquide (R), est envoyée dans une section de fractionnement, pour séparer une fraction liquide d'hydrocarbures constitués de paraffines linéaires comportant au plus 17 atomes de carbone (fraction C17-), et une fraction liquide d'hydrocarbures constitués de paraffines linéaires comportant 18 atomes de carbone ou plus (C18+).

13. Procédé selon la revendication 12 dans lequel seules les paraffines linéaires comportant au plus 17 atomes de carbone sont envoyées dans l'étape b) de transformation du procédé selon l'invention.

14. Procédé selon l'une des revendications 1 à 11 dans lequel ladite étape b) est :

    - soit une étape d'alkylation d'au moins une partie de ladite fraction liquide d'hydrocarbures constitués de paraffines linéaires issue de l'étape a) et de préférence d'une fraction liquide d'hydrocarbures constitués de paraffines linéaires comportant au plus 17 atomes de carbone (fraction C17-), par des hydrocarbures aromatiques choisis parmi le benzène, le toluène, les xylènes ou des mélanges de ces hydrocarbures, pour produire des composés aromatiques alkylés par des chaînes paraffiniques longues,

    - soit une étape de sulfonation d'au moins une partie de ladite fraction liquide d'hydrocarbures constitués de paraffines linéaires issue de l'étape a) et de préférence d'une fraction liquide d'hydrocarbures constitués de paraffines linéaires comportant au plus 17 atomes de carbone (fraction C17-), pour produire des composés sulfonates paraffiniques.

**Patentansprüche**

1. Verfahren zur Herstellung von Tensidverbindungen aus einer Charge, die aus erneuerbaren Quellen stammt, das mindestens die folgenden Schritte umfasst:

    a) einen Schritt zur Hydrobehandlung der Charge zum Erzeugen eines Abstroms, der mindestens Kohlenwasserstoffverbindungen enthält, die aus linearen Paraffinen bestehen, in Gegenwart eines Wasserstoffüberschusses, im Hinblick auf den theoretischen Wasserstoffverbrauch, und unter Hydrobehandlungsbedingungen in einem Festbettreaktor, der mehrere, in Reihe angeordnete und einen Hydrobehandlungskatalysator umfassende katalytische Zonen aufweist, wobei:

        i) der Strom der Gesamtcharge F in eine bestimmte Anzahl von verschiedenen Teilströmen F1 bis Fn gleich der Anzahl der katalytischen Zonen n in dem Reaktor geteilt wird, wobei der erste Teilstrom F1 in die erste katalytische Zone injiziert wird, der zweite Teilstrom F2 in die zweite katalytische Zone injiziert wird und so weiter, wenn n größer 2 ist,
        die verschiedenen Teilströme in die aufeinander folgenden katalytischen Zonen in steigenden Anteilen derart injiziert werden, dass F1/F kleiner F2/F ist, dieses selbst kleiner F3/F ist und so weiter, bis F(n-1)/F kleiner Fn/F ist,
        die Temperatur des am Eingang der ersten katalytischen Zone Z1 injizierten Stroms, umfassend den Teilstrom der Charge F1 gemischt mit dem wasserstoffreichen Gasstrom H1, der in die Zone Z1 eintritt, und mit einer flüssigen Recyclingfraktion R, (F1 + R + H1), größer als 250°C ist, und die Temperatur der Ströme am Eingang der folgenden katalytischen Zonen Z2 bis Zn größer als 300°C ist,

ii) wobei der Abstrom, der mindestens Kohlenwasserstoffverbindungen enthält, die aus linearen Paraffinen bestehen, mindestens einem Trennschritt unterzogen wird, der es ermöglicht, eine gasförmige Fraktion, die Wasserstoff, CO, $CO_2$, $H_2S$, Wasser und die leichten Gase enthält, und eine flüssige Kohlenwasserstofffraktion, die aus linearen Paraffinen besteht, zu trennen,

iii) mindestens ein Teil R der flüssigen Kohlenwasserstofffraktion, die aus linearen Paraffinen besteht, derart in die erste katalytische Zone Z1 recycelt wird, dass das Masseverhältnis zwischen dem Strom der Recyclingfraktion R und dem Teilstrom F1, der in die erste katalytische Zone Z1 eingeführt wird, R/F1, größer oder gleich 8 ist,

b) einen Schritt zum Umwandeln mindestens eines Teils der die aus Schritt a) stammenden flüssigen Kohlenwasserstofffraktion, die aus linearen Paraffinen besteht, in Tensidverbindungen.

2. Verfahren nach Anspruch 1, wobei der im Hydrobehandlungsschritt verwendete Wasserstoffüberschuss mindestens 50 % des theoretischen Verbrauchs beträgt.

3. Verfahren nach einem der Ansprüche 1 oder 2, wobei das Masseverhältnis zwischen dem Strom der Recyclingfraktion, die in die erste katalytische Zone geschickt wird, und dem Gesamtstrom der Charge kleiner 1, bevorzugt kleiner 0,5 ist.

4. Verfahren nach einem der Ansprüche 1 bis 3, wobei die Charge, die aus erneuerbaren Quellen stammt, ausgewählt ist aus Ölen und Fetten pflanzlichen oder tierischen Ursprungs oder Gemischen solcher Chargen, die Triglyceride und/oder freie Fettsäuren und/oder Ester enthalten.

5. Verfahren nach einem der Ansprüche 1 bis 4, wobei die Temperatur des am Eingang der ersten katalytischen Zone Z1 injizierten Stroms, das heißt, der Charge gemischt mit der flüssigen Recyclingfraktion, bestehend aus einem Teil der flüssigen Kohlenwasserstofffraktion, die aus linearen Paraffinen besteht, größer als 260 °C ist.

6. Verfahren nach Anspruch 5, wobei die Temperatur des am Eingang der ersten katalytischen Zone Z1 injizierten Stroms, das heißt, der Charge gemischt mit der flüssigen Recyclingfraktion, bestehend aus einem Teil der flüssigen Kohlenwasserstofffraktion, die aus linearen Paraffinen besteht, größer als 270°C ist.

7. Verfahren nach einem der Ansprüche 1 bis 6, wobei die Temperatur des Stroms am Eingang der katalytischen Zonen Z2 bis Zn größer als 310°C ist.

8. Verfahren nach einem der Ansprüche 1 bis 7, wobei die Hydrobehandlung bei einer Temperatur im Bereich zwischen 200 und 400°C, bei einem Gesamtdruck im Bereich zwischen 2 MPa und 15 MPa, bei einer Raumgeschwindigkeit pro Stunde im Bereich zwischen 0,1 $h^{-1}$ und 5 $h^{-1}$ und in Gegenwart einer Wasserstoffgesamtmenge, die derart mit der Charge gemischt ist, dass das Verhältnis Wasserstoff/Charge im Bereich zwischen 300 und 1.500 $Nm^3$ Wasserstoff/$m^3$ Charge liegt, durchgeführt wird.

9. Verfahren nach einem der Ansprüche 1 bis 8, wobei der Hydrobehandlungskatalysator ein sulfurierter Katalysator ist, der ein oder mehrere Elemente der Gruppen 6, 8, 9 und 10 des Periodensystems, bevorzugt ausgewählt aus Nickel, Molybdän, Wolfram und Cobalt, einzeln oder als Gemisch, und einen Träger, ausgewählt aus der Gruppe bestehend aus Aluminiumoxid, Siliciumdioxid, Siliciumdioxid-Aluminiumoxiden, Magnesia, Tonen und dem Gemisch mindestens zweier dieser Mineralien, umfasst.

10. Verfahren nach einem der Ansprüche 1 bis 8, wobei der Hydrobehandlungskatalysator ein Metallkatalysator ist, der ein Metall umfasst, ausgewählt aus Nickel, Platin, Palladium, Ruthenium und Rhodium, geträgert auf einem Träger, ausgewählt aus Aluminiumoxid, Siliciumdioxid, Siliciumdioxid-Aluminiumoxid, Kohlenstoff, Aktivkohle, Ceroxid und Zircon oder aus einem Gemisch dieser Verbindungen.

11. Verfahren nach einem der Ansprüche 1 bis 10, wobei mindestens ein Teil der aus dem Trennschritt stammenden flüssigen Kohlenwasserstofffraktion, die aus linearen Paraffinen besteht, derart in die erste katalytische Zone Z1 recycelt wird, dass das Masseverhältnis zwischen dem Strom der Recyclingfraktion und dem Teilstrom F1, der in die erste katalytische Zone Z1 eingeführt wird, größer oder gleich 10 ist.

12. Verfahren nach einem der Ansprüche 1 bis 11, wobei der Teil der aus dem Trennschritt stammenden flüssigen Kohlenwasserstofffraktion, die aus linearen Paraffinen besteht, nicht recycelt wird, um zum Einsatzstrom gegeben zu werden, der als flüssige Recyclingfraktion (R) in die Zone Z1 injiziert wird, sondern in einen Fraktionierungsabschnitt geschickt wird, um eine flüssige Kohlenwasserstofffraktion, die aus linearen Paraffinen besteht, die höchstens 17 Kohlenstoffatome (Fraktion C17-) umfassen, und eine flüssige Kohlenwasserstofffraktion, die aus linearen Paraffinen besteht, die 18 Kohlenstoffatome oder mehr (C18+) umfassen, zu trennen.

13. Verfahren nach Anspruch 12, wobei nur die linearen Paraffine, die höchstens 17 Kohlenstoffatome umfassen, in den Umwandlungsschritt b) des Verfahrens gemäß der Erfindung geschickt werden.

14. Verfahren nach einem der Ansprüche 1 bis 11, wobei der Schritt b) aus Folgendem besteht:

- entweder einem Schritt zum Alkylieren mindestens eines Teils der aus Schritt a) stammenden flüssigen Kohlenwasserstofffraktion, die aus linearen Paraffinen besteht, und bevorzugt einer flüssigen Kohlenwasserstofffraktion, die aus linearen Paraffinen besteht, die höchstens 17 Kohlenstoffatome umfassen (Fraktion C17-), durch aromatische Kohlenwasserstoffe, ausgewählt aus Benzol, Toluol, Xylolen oder Gemischen dieser Kohlenwasserstoffe, um mit langen Paraffinketten alkylierte aromatische Verbindungen zu erzeugen,
- oder einem Schritt zum Sulfonieren mindestens eines Teils der aus Schritt a) stammenden flüssigen Kohlenwasserstofffraktion, die aus linearen Paraffinen besteht, und bevorzugt einer flüssigen Kohlenwasserstofffraktion, die aus linearen Paraffinen besteht, die höchstens 17 Kohlenstoffatome umfasst (Fraktion C17-), um Paraffinsulfonatverbindungen zu erzeugen.

**Claims**

1. A process for the production of surfactant compounds from a feed obtained from renewable sources, comprising at least the following steps:

a) a step for hydrotreatment of said feed in order to produce an effluent containing at least hydrocarbon compounds constituted by linear paraffins, in the presence of hydrogen in excess of the theoretical hydrogen consumption and under hydrotreatment conditions, in a fixed bed reactor having a plurality of catalytic zones disposed in series and comprising a hydrotreatment catalyst, in which:

i) the total flow of feed F is divided into a certain number of different part flows, F1 to Fn, equal to the number of catalytic zones n in the reactor, the first part flow F1 is injected into the first catalytic zone, the second part flow F2 is injected into the second catalytic zone and so on, if n is greater than 2,

the various part flows being injected into the successive catalytic zones in increasing proportions such that F1/F is less than to F2/F, which itself is less than to F3/F and so on until F(n-1)/F is less than to Fn/F, the temperature of the flow injected into the inlet to the first catalytic zone Z1 comprising the part flow of the feed F1 mixed with the hydrogen-rich flow H1 entering the zone Z1 and with a liquid recycle R, (F1 + R + HI), being greater than 250°C and the temperature of the flows at the inlet to the subsequent zones Z2 to Zn being greater than 300°C;

ii) said effluent containing at least the hydrocarbon compounds constituted by linear paraffins undergoes at least one separation step in order to separate a gaseous fraction containing hydrogen, CO, $CO_2$, $H_2S$, water and light gases and a liquid hydrocarbon fraction constituted by linear paraffins;

iii) at least a portion R of said liquid hydrocarbon fraction constituted by linear paraffins is recycled to the first catalytic zone Z1 such that the weight ratio between the flow of said recycle R and the part flow F1 introduced into the first catalytic zone Z1, R/F1, is 8 or more;

b) a step for transforming at least a portion of said liquid hydrocarbon fraction constituted by linear paraffins obtained from step a) into surfactant compounds.

2. The process according to claim 1, in which the excess hydrogen used in the hydrotreatment step is at least 50% of the theoretical consumption.

3. The process according to claim 1 or claim 2, in which the weight ratio between the recycle flow sent to the first catalytic zone and the total flow of feed is less than 1, preferably less than 0.5.

4. The process according to one of claims 1 to 3, in which the feed obtained from renewable sources is selected from oils and fats of vegetable or animal origin, or mixtures of such feeds, containing triglycerides and/or free fatty acids and/or esters.

5. The process according to one of claims 1 to 4, in which the temperature of the flow injected into the inlet to the first catalytic zone Z1, i.e. the feed mixed with the liquid recycle constituted by a portion of the liquid hydrocarbon fraction constituted by linear paraffins, is greater than 260°C.

6. The process according to claim 5, in which the temperature of the flow injected into the inlet to the first catalytic zone Z1, i.e. the feed mixed with the liquid recycle constituted by a portion of the liquid hydrocarbon fraction constituted by linear paraffins, is greater than 270°C.

7. The process according to one of claims 1 to 6, in which the temperature of the flows at the inlet to the catalytic zones Z2 to Zn is greater than 310°C.

8. The process according to one of claims 1 to 7, in which the hydrotreatment is operated at a temperature in the range 200°C to 400°C, a total pressure in the range 2 MPa to 15 MPa, at an hourly space velocity in the range $0.1 \, h^{-1}$ to $5 \, h^{-1}$ and in the presence of a total quantity of hydrogen mixed with the feed such that the hydrogen/feed ratio is in the range 300 to 1500 $Nm^3$ of hydrogen/$m^3$ of feed.

9. The process according to one of claims 1 to 8, in which the hydrotreatment catalyst is a sulphur-containing catalyst comprising one or more elements from groups 6, 8, 9 and 10 of the periodic classification of the elements, preferably selected from nickel, molybdenum, tungsten and cobalt, used alone or as a mixture, and a support selected from the group formed by alumina, silica, silica-aluminas, magnesia, clays and mixtures of at least two of these minerals.

10. The process according to one of claims 1 to 8, in which the hydrotreatment catalyst is a metallic catalyst which comprises a metal selected from nickel, platinum, palladium, ruthenium and rhodium, supported on a support selected from alumina, silica, silica-alumina, carbon, activated carbon, cerine and zirconia or a mixture of these compounds.

11. The process according to one of claims 1 to 10, in which at least a portion of the liquid hydrocarbon fraction constituted by linear paraffins obtained from the separation step is recycled to the first catalytic zone Z1 such that the weight ratio between the flow for said recycle and the part flow F1 introduced into the first catalytic zone Z1 is greater than or equal to 10.

12. The process according to one of claims 1 to 11, in which the portion of the liquid hydrocarbon fraction constituted by linear paraffins obtained from the separation step which is not recycled to supplement the flow of feed injected into the zone Z1 as a liquid recycle (R) is sent to a fractionation section in order to separate a liquid hydrocarbon fraction constituted by linear paraffins containing at most 17 carbon atoms (C17- fraction) and a liquid hydrocarbon fraction constituted by linear paraffins containing 18 carbon atoms or more (C18+).

13. The process according to claim 12, in which only the linear paraffins containing at most 17 carbon atoms are sent to the step b) for transformation in accordance with the process of the invention.

14. The process according to one of claims 1 to 11, in which said step b) is:

   • either a step for alkylation of at least a portion of said liquid hydrocarbon fraction constituted by linear paraffins obtained from step a), preferably a liquid hydrocarbon fraction constituted by linear paraffins containing at most 17 carbon atoms (C17- fraction), by aromatic hydrocarbons selected from benzene, toluene, xylenes or mixtures of these hydrocarbons, in order to produce aromatic compounds alkylated by long chain paraffins;
   • or a step for sulphonation of at least a portion of said liquid hydrocarbon fraction constituted by linear paraffins obtained from step a), preferably a liquid hydrocarbon fraction constituted by linear paraffins containing at most 17 carbon atoms (C17-fraction), in order to produce paraffinic sulphonate compounds.

Figure 1

# Figure 2

**EP 2 864 448 B2**

## RÉFÉRENCES CITÉES DANS LA DESCRIPTION

**Documents brevets cités dans la description**

- US 20070281875 A
- EP 1741768 A
- US 7070745 B
- US 2011275871 A